# EUROPÄISCHE PATENTSCHRIFT

(11) **EP 2 604 185 B1**
(45) Veröffentlichungstag und Bekanntmachung des Hinweises auf die Patenterteilung: **25.09.2019**
(21) Anmeldenummer: 12008411.6
(22) Anmeldetag: 18.12.2012
(51) Int. Cl.: A61B 5/12

(54) **Vorrichtung und Verfahren zur Erfassung von distorsiv produzierten otoakustischen Emissionen unter Berücksichtung von technisch bedingten Verzerrungsprodukten**
Device and method for detecting distortion product otoacoustic emissions taking into account technical distortion products
Dispositif et procédé de détermination d'oto-émissions acoustiques produites par distorsion en tenant compte de produits de déformation imposés par la technologie

(30) Priorität: 18.12.2011 DE 102011121686
(43) Veröffentlichungstag der Anmeldung: 19.06.2013
(73) Patentinhaber: Path Medical GmbH, 82110 Germering (DE)
(72) Erfinder: Lodwig, Andre, 82237 Wörthsee (DE); Janssen, Thomas, 83104 Tuntenhausen (DE); Oswald, Hans, 85567 Grafing (DE); Zoth, Peter, 82205 Gilching (DE)
(74) Vertreter: Zimmermann & Partner Patentanwälte mbB

(56) Entgegenhaltungen:
- US-A- 5 811 681
- US-A1- 2004 037 428

## Beschreibung

Die Erfindung betrifft eine Vorrichtung zur Erfassung von distorsiv produzierten otoakustischen Emissionen gemäß der im Oberbegriff des Anspruchs 1 näher angegebenen Art.

Zum Stand der Technik wird auf die Patentschrift US 2004/0037428 A1 hingewiesen, aus der eine Vorrichtung (und ein zugehöriges Verfahren) zur Audiometrie bekannt ist, mit einer Ohrsonde zur Aufnahme des Schalls im äußeren Gehörgang und mit einer Auswerteeinrichtung, mit der der mit der Ohrsonde aufgenommene Schall ausgewertet werden kann.

Otoakustische Emissionen sind Schallemissionen, deren Ursprung im Innenohr (in der Cochlea) liegt und die von dort über das Mittelohr und den Trommelfell-Gehörknöchelchen-Apparat in den äußeren Gehörgang übertragen werden, wo sie mit einem Mikrofon oder dergleichen aufgenommen werden können. Otoakustische Emissionen können zum Hörtest und zum Hörscreening herangezogen werden.

Bei distorsiv produzierten otoakustischen Emissionen (auch als distorsivproduzierte otoakustische Emissionen oder DPOAE bezeichnet) handelt es sich um eine spezielle Form otoakustischer Emissionen, die dann in Erscheinung treten, wenn eine Anregung bzw. Stimulation des Gehörapparats mit zwei Stimulationstönen, deren Frequenzen in einem bestimmten Verhältnis zueinander stehen, erfolgt, woraufhin im Innenohr eine dritte Schwingung mit einer anderen Frequenz (so genanntes Verzerrungsprodukt) erzeugt wird, welche dann wiederum im äußeren Gehörgang aufgenommen werden kann. Im Weiteren wird auf die entsprechende Fachliteratur verwiesen.

Die Erfassung von distorsiv produzierten otoakustischen Emissionen kann mit einer Vorrichtung gemäß den Merkmalen des Oberbegriffs des Anspruchs 1 erfolgen. Unter einem Erfassen von distorsiv produzierten otoakustischen Emissionen kann hierin allgemein und in nicht einschränkender Weise die Aufnahme und die nachfolgende Auswertung solcher distorsiv produzierten otoakustischen Emissionen verstanden werden.

Problematisch ist jedoch, dass die technischen Komponenten einer solchen Vorrichtung, insbesondere aufgrund nicht linearer Kennlinien, technisch bedingte Verzerrungen generieren, die zu Verzerrungsprodukten führen, welche dann fälschlicherweise als das Ergebnis von otoakustischen Emissionen interpretiert werden können.

Die Aufgabe der Erfindung ist es, hier Abhilfe zu schaffen.

Diese Aufgabe wird gelöst durch eine erfindungsgemäße Vorrichtung mit den Merkmalen des Anspruchs 1. Bevorzugte Weiterbildungen und Ausgestaltungen ergeben sich aus den abhängigen Ansprüchen sowie auch aus den nachfolgenden Erläuterungen.

Mit einem nebengeordneten Anspruch erstreckt sich die Lösung der Aufgabe auch auf ein erfindungsgemäßes Verfahren zur Erfassung von distorsiv produzierten otoakustischen Emissionen und insbesondere zur Stimulation und Erfassung von distorsiv produzierten otoakustischen Emissionen.

Die vorausgehenden und/oder nachfolgenden Erläuterungen gelten analog für alle Erfindungsgegenstände. Diese gilt ausdrücklich auch für die beschriebenen und/oder in den Figuren gezeigten Weiterbildungen und Ausgestaltungen.

Die erfindungsgemäße Vorrichtung dient der Erfassung von distorsiv produzierten otoakustischen Emissionen, insbesondere an einem menschlichen Ohr. Die erfindungsgemäße Vorrichtung kann aber auch der Erfassung von distorsiv produzierten otoakustischen Emissionen an einem tierischen Ohr und insbesondere an dem Ohr eines Land-Wirbeltiers dienen.

Die erfindungsgemäße Vorrichtung umfasst:
- wenigstens eine Ohrsonde, die in den äußeren Gehörgang eingesetzt werden kann bzw. einsetzbar ist oder an den äußeren Gehörgang angesetzt werden kann bzw. ansetzbar ist und mit der der im äußeren Gehörgang vorhandene Schall (Gesamtschall) aufgenommen werden kann; und
- wenigstens eine Auswerteeinrichtung, mit der der mit der Ohrsonde aufgenommene Schall im Hinblick auf distorsiv produzierte otoakustische Emissionen ausgewertet werden kann.

Erfindungsgemäß ist nun vorgesehen, dass die zur erfindungsgemäßen Vorrichtung gehörende Auswerteeinrichtung die Fähigkeit aufweist, im aufgenommenen Schall das Vorhandensein und/oder das Nicht-Vorhandensein technisch bedingter Verzerrungsprodukte , im aufgenommenen Schall, welche fälschlicherweise als das Ergebnis von otoakustischen Emissionen interpretiert werden können, automatisch zu erkennen und hierauf zu reagieren bzw. wenigstens eine Reaktion herbeizuführen.

Ein Reagieren kann ein direktes bzw. unmittelbares Herbeiführen wenigstens einer Reaktion durch die Auswerteeinrichtung sein. Ein Reagieren kann jedoch auch ein indirektes bzw. mittelbares Herbeiführen wenigstens einer Reaktion sein, wobei die betreffende Reaktion von der bzw. durch die Auswerteeinrichtung lediglich initiiert wird.

Die zur erfindungsgemäßen Vorrichtung gehörende Auswerteeinrichtung kann in einem Messgerät, das mit der Ohrsonde verbunden bzw. verbindbar ist, untergebracht sein. Dieses Messgerät kann weitergehende Funktionen (bspw. die Steuerung oder Regelung des gesamten Messdurchlaufs) umfassen.

Das Erzeugen von wenigstens zwei Stimulationstönen kann bekanntermaßen auf sehr verschiedene Weisen erfolgen. Beide Stimulationstöne (oder zumindest einer der Stimulationstöne) können z. B. außerhalb des Ohrs erzeugt und durch einen Schlauch (oder dergleichen) oder mechanisch (bspw. als Körper- und insbesondere Knochenschall über den Schädel) in das zu untersuchende Ohr eingeleitet bzw. eingekoppelt werden. Ferner können die Stimulationstöne (oder zumindest einer der Stimulationstöne) auch in der Ohrsonde (gegebenenfalls auch mit mehreren Ohrsonden) erzeugt und auf diese Weise in das Ohr eingeleitet bzw. eingekoppelt werden.

Bevorzugt ist vorgesehen, dass es sich bei der erfindungsgemäßen Vorrichtung um eine Vorrichtung zur Stimulation und Erfassung von distorsiv produzierten otoakustischen Emissionen handelt. Eine solche Vorrichtung umfasst:
- wenigstens eine Ohrsonde bzw. Gehörgangsonde, die in den äußeren Gehörgang eingesetzt werden kann bzw. einsetzbar ist oder die an den äußeren Gehörgang angesetzt werden kann bzw. ansetzbar ist und mit der simultan wenigstens zwei Stimulationstöne erzeugt werden können und mit der der zeitgleich (oder unmittelbar danach) im äußeren Gehörgang vorhandene Schall (Gesamtschall) aufgenommen bzw. registriert werden kann; und
- ein Messgerät, das mit der Ohrsonde verbunden oder verbindbar ist und mit dem die Ohrsonde angesteuert und der aufgenommene Schall im Hinblick auf die distorsiv produzierten otoakustischen Emissionen ausgewertet und insbesondere automatisiert ausgewertet werden kann.

Erfindungsgemäß ist nun vorgesehen, dass das zu der erfindungsgemäßen Vorrichtung gehörende Messgerät die Fähigkeit aufweist, das Vorhandensein und/oder das Nicht-Vorhandensein technisch bedingter Verzerrungsprodukte (welche fälschlicherweise als das Ergebnis von otoakustischen Emissionen interpretiert werden können) im aufgenommenen Schall automatisch zu erkennen und hierauf zu reagieren bzw. wenigstens eine Reaktion herbeizuführen. Insbesondere ist vorgesehen, dass das Messgerät wenigstens eine Auswerteeinrichtung aufweist, die über eine solche Funktionalität verfügt.

Das Erkennen von vorhandenen oder auch nicht vorhandenen technisch bedingten Verzerrungsprodukten erfolgt insbesondere beim Auswerten des aufgenommenen Schalls. Unter Auswerten kann hierin allgemein und in nicht einschränkender Weise das Aufbereiten des aufgenommenen Schalls zur Herbeiführung eines ausgebbaren bzw. anzeigbaren Ergebnisses (das insbesondere Rückschlüsse auf die physiologische Funktionsfähigkeit des untersuchten Ohrs erlaubt) verstanden werden.

Demnach kann die Auswerteeinrichtung und/oder das Messgerät der erfindungsgemäßen Vorrichtung mit der Funktionalität ausgestattet sein, beim Auswerten des aufgenommenen Schalls technisch bedingte Verzerrungsprodukte, die bspw. aus den nicht linearen Kennlinienverläufen der tonerzeugenden Lautsprecher und/oder aus dem nicht linearen Kennlinienverlauf des schallaufnehmenden Mikrofons resultieren können, und die von den physiologisch bzw. biomechanischen Verzerrungsprodukten zu unterscheiden sind, im Zuge der Auswertung automatisch als solche zu erkennen.

Alternativ oder ergänzend hierzu kann die Auswerteeinrichtung und/oder das Messgerät der erfindungsgemäßen Vorrichtung aber auch mit der Funktionalität ausgestattet sein, beim Auswerten des aufgenommenen Schalls entweder das Nicht-Vorhandensein bzw. die Nicht-Existenz technisch bedingter Verzerrungsprodukte (welche fälschlicherweise als das Ergebnis von otoakustischen Emissionen interpretiert werden können) zu erkennen und/oder zumindest das Vorhandensein bzw. die Existenz technisch bedingter Verzerrungsprodukte (welche fälschlicherweise als das Ergebnis von otoakustischen Emissionen interpretiert werden können; insbesondere oberhalb einer kritischen Schwelle) mit hoher Wahrscheinlichkeit auszuschließen und dadurch die Auswertung und das Ergebnis der Auswertung glaubhaft zu machen.

Die Auswerteeinrichtung und/oder das Messgerät der erfindungsgemäßen Vorrichtung weist die Fähigkeit auf bzw. ist mit der Funktionalität ausgestattet, beim Erkennen von vorliegenden oder auch nicht vorliegenden technisch bedingten Verzerrungsprodukten (welche fälschlicherweise als das Ergebnis von otoakustischen Emissionen interpretiert werden können) wenigstens eine geeignete Reaktion bzw. Maßnahme herbeiführen zu können. Das Herbeiführen einer Reaktion kann direkt bzw. unmittelbar durch die Auswerteeinrichtung und/oder das Messgerät erfolgen. Das Herbeiführen einer Reaktion kann aber auch mittelbar erfolgen, in dem Sinne, dass die Auswerteeinrichtung und/oder das Messgerät eine Reaktion initiiert bzw. veranlasst, wobei die Reaktion im Weiteren durch wenigstens eine andere Einrichtung herbeigeführt und/oder ausgeführt wird.

Wird das Nicht-Vorhandensein technisch bedingter Verzerrungsprodukte (welche fälschlicherweise als das Ergebnis von otoakustischen Emissionen interpretiert werden können) erkannt, so kann z. B. als Reaktion am Messgerät und/oder an der Ohrsonde eine optische und/oder akustische Meldung ausgegeben werden ("Messung O.K."), wozu das Messgerät und/oder die Ohrsonde mit einer entsprechenden Einrichtung ausgestattet ist. D. h. das Messgerät und/oder die Ohrsonde kann wenigstens eine Anzeigeeinrichtung aufweisen, über die eine optische und/oder akustische Meldung ausgegeben werden kann, wie nachfolgend noch näher erläutert. Die Reaktion auf das Nicht-Vorhandensein technisch bedingter Verzerrungsprodukte (welche fälschlicherweise als das Ergebnis von otoakustischen Emissionen interpretiert werden können) kann jedoch auch darin liegen, dass die Auswertung automatisch fortgesetzt und das Ergebnis der Auswertung ausgegeben wird, ohne dass hierbei eine gesonderte Meldung oder dergleichen erfolgt.

Wie bereits obenstehend erläutert, kann die Auswerteeinrichtung in einem Messgerät integriert sein. Die Auswerteeinrichtung kann aber auch in der Ohrsonde (d. h. insbesondere im Gehäuse der Ohrsonde) integriert sein. Ebenso kann vorgesehen sein, dass die Ohrsonde und das Messgerät einteilig ausgebildet und bspw. in einem gemeinsamen Gehäuse zusammengefasst sind. In diesem Fall ist besonders bevorzugt vorgesehen, dass die Ohrsonde mit wenigstens einer Anzeigeeinrichtung ausgestattet ist.

Wird von der Auswerteeinrichtung das Vorhandensein technisch bedingter Verzerrungsprodukte erkannt (welche fälschlicherweise als das Ergebnis von otoakustischen Emissionen interpretiert werden können), so können z. B. die folgenden zweckmäßigen Reaktionen veranlasst werden, was insbesondere automatisch erfolgt. Bevorzugt ist zudem vorgesehen, dass eine Reaktion erst dann herbeigeführt bzw. veranlasst wird, wenn die erkannten technisch bedingten Verzerrungsprodukte eine vorgegebene oder auch einstellbare Schwelle erreichen, über- und/oder unterschreiten.

Eine bevorzugte Reaktion beim Erkennen technisch bedingter Verzerrungsprodukte ist das Ausgeben einer Warnmeldung. Die Warnmeldung kann z. B. über ein Display oder wenigstens eine Leuchtanzeige oder dergleichen ausgeben werden. Ferner ist auch das Ausgeben einer akustischen Warnmeldung denkbar. Ergänzend kann als Reaktion vorgesehen sein, dass die Auswertung abgebrochen wird.

Eine andere bevorzugte Reaktion beim Erkennen technisch bedingter Verzerrungsprodukte ist das Fortsetzen des Messdurchlaufs oder Wiederholen des Messdurchlaufs insbesondere mit geänderten Parametern (bspw. veränderte Frequenz und/oder Pegel für die Stimulationstöne). Hierbei kann vorgesehen sein, dass nur eine vorgegebene Anzahl von neuen Messdurchläufen gestartet wird und dass dann eine Meldung ausgegeben wird, falls hierbei kein zuverlässiges bzw. glaubhaftes Ergebnis erzielt werden konnte.

Eine weitere bevorzugte Reaktion beim Erkennen technisch bedingter Verzerrungsprodukte ist das zumindest teilweise Unterdrücken dieser technisch bedingten Verzerrungsprodukte. Somit könnte trotz Vorhandensein technisch bedingter Verzerrungsprodukte (welche fälschlicherweise als das Ergebnis von otoakustischen Emissionen interpretiert werden können) ein zumindest teilweise bereinigtes bzw. korrigiertes Ergebnis ausgegeben werden. In der Auswerteeinrichtung, im Messgerät oder sonst wo in der erfindungsgemäßen Vorrichtung kann bspw. ein mathematisches Modell für technische Komponenten (damit ist wenigstens eine technische Komponenten gemeint), insbesondere für die technischen Komponenten der Ohrsonde (dies sind die tonerzeugenden Lautsprecher und das schallaufnehmende Mikrofon), hinterlegt sein, welches die Nicht-Linearitäten dieser Komponenten abbildet und hiermit die rechnerische Unterdrückung technisch bedingter Verzerrungsprodukte ermöglicht. Ein solches mathematisches Modell kann bspw. als Softwarecode in einem elektronischen Datenträger irgendwo in der erfindungsgemäßen Vorrichtung hinterlegt sein.

Ferner ist bevorzugt vorgesehen, dass die Auswerteeinrichtung und/oder das Messgerät der erfindungsgemäßen Vorrichtung die Fähigkeit bzw. Funktionalität aufweist, unterschiedliche Reaktionen (bspw. der zuvor erläuterten Art) herbeiführen oder veranlassen zu können, falls technisch bedingte Verzerrungsprodukte im aufgenommenen Schall erkannt oder auch nicht erkannt werden (im Sinne von Vorhandensein und Nicht-Vorhandensein), wobei eine herbeizuführende Reaktion oder gegebenenfalls auch mehrere herbeizuführende Reaktionen durch Auswahl voreinstellbar ist bzw. sind. Ferner ist denkbar, dass das Herbeiführen von Reaktionen wahlweise auch ausschaltbar ist.

Die Auswerteeinrichtung und/oder das Messgerät der erfindungsgemäßen Vorrichtung kann die Fähigkeit bzw. die Funktionalität aufweisen, das Vorhandensein von technisch bedingten Verzerrungsprodukten (welche fälschlicherweise als das Ergebnis von otoakustischen Emissionen interpretiert werden können) durch Analyse des Spektrums bzw. der spektralen Zusammensetzung des aufgenommenen Schalls zu erkennen bzw. zu detektieren. Im Rahmen der Auswertung kann z. B. das Spektrum des aufgenommenen Schalls bestimmt bzw. abgeleitet und dann betrachtet bzw. analysiert werden. Bestimmte Anteile im Spektrum können dann einen Hinweis darauf geben, ob technisch bedingte Verzerrungsanteile im Spektrum vorhanden sind, woraus auf das Vorhandensein von technisch bedingten Verzerrungsprodukten (welche fälschlicherweise als das Ergebnis von otoakustischen Emissionen interpretiert werden können) geschlossen werden kann.

Die erfindungsgemäße Vorrichtung kann wenigstens einen Datenträger mit einem darauf abgespeicherten Programmcode (Software) und wenigstens einen Prozessor (zur Auswertung und Ausführung des Programmcodes) aufweisen, um zumindest die wesentlichen Funktionen der Auswerteeinrichtung und/oder des Messgeräts automatisiert bzw. selbsttätig zu steuern bzw. ausführen zu können. Dieser Datenträger kann in der Auswerteeinrichtung und/oder im Messgerät oder sonst wo in der erfindungsgemäßen Vorrichtung angeordnet sein. Wesentliche Funktionen bzw. Funktionalitäten der Auswerteeinrichtung sind z. B. das Auswerten des aufgenommenen Schalls, das Erkennen von vorhandenen oder auch nicht vorhandenen technisch bedingten Verzerrungsprodukten (welche fälschlicherweise als das Ergebnis von otoakustischen Emissionen interpretiert werden können) im aufgenommenen Schall und/oder das Herbeiführen wenigstens einer Reaktion wie bspw. das Unterdrücken von technisch bedingten Verzerrungsprodukten (welche fälschlicherweise als das Ergebnis von otoakustischen Emissionen interpretiert werden können). Wesentliche Funktionen bzw. Funktionalitäten des Messgeräts sind z. B. die vorausgehend genannten Funktionen bzw. Funktionalitäten sowie auch das Ausführen eines Messdurchlaufs. Bevorzugt werden alle diese Funktionen bzw. Funktionalitäten mittels Programmcode und Prozessor automatisiert ausgeführt. Demnach handelt es sich bei dem zur erfindungsgemäßen Vorrichtung gehörenden Auswerteeinrichtung und/oder Messgerät um eine softwaregesteuerte Auswerteeinrichtung bzw. um ein softwaregesteuertes Messgerät.

Das erfindungsgemäße Verfahren dient der Erfassung von distorsiv produzierten otoakustischen Emissionen und insbesondere der Stimulation und Erfassung von distorsiv produzierten otoakustischen Emissionen. Dieses Verfahren umfasst zumindest die folgenden Schritte, die zumindest teilweise und vorzugsweise im Gesamten automatisiert ausgeführt werden können:
- gegebenenfalls Stimulation von distorsiv produzierten otoakustischen Emissionen;
- Aufnehmen des im äußeren Gehörgang (des zu untersuchenden Ohrs) vorhandenen Schalls;
- Auswerten des aufgenommen Schalls im Hinblick auf distorsiv produzierte otoakustische Emissionen; und
- Herbeiführen einer Reaktion, falls beim Auswerten im aufgenommenen Schall das Vorhandensein und/oder das Nicht-Vorhandensein technisch bedingter Verzerrungsprodukte, welche fälschlicherweise als das Ergebnis von otoakustischen Emissionen interpretiert werden können, erkannt wird oder eine solches Vorhandensein und/oder Nicht-Vorhandensein zumindest mit hoher Wahrscheinlichkeit angenommen oder ausgeschlossen werden kann.

Das beanspruchte Verfahren soll nur zur Messung und/oder Aufzeichnung von Eigenschaften menschlicher oder tierischer Körper dienen. Das beanspruchte Verfahren umfasst demnach nur die benannten technischen Maßnahmen zur Feststellung eines physikalischen bzw. physiologischen Zustands, ohne jegliche medizinische schlussfolgernde Wertung.

Die Auswertung des aufgenommenen Schalls erfolgt sozusagen unter Berücksichtigung bzw. Beachtung technisch bedingter Verzerrungsprodukte. Hierbei ist vorzugsweise vorgesehen, dass etwaige technisch bedingten Verzerrungen nicht einfach pauschal berücksichtigt werden (bspw. auf Grundlage von vorausgehenden Testmessungen oder dergleichen), sondern dass insbesondere das Vorhandensein und/oder Nicht-Vorhandensein technisch bedingter Verzerrungsprodukte beim Auswerten individuell (bzw. spezifisch) anhand (bzw. auf Basis oder auf Grundlage) des betreffenden (bzw. jeweils auszuwertenden) aufgenommenen Schalls erkannt wird (bzw. erkannt werden soll), um dann hierauf reagieren zu können. Hierbei kann dann auch berücksichtigt werden, dass sich jedes Ohr akustisch anders verhält, was wiederum Einfluss auf die technisch bedingten Verzerrungen haben kann.

Das erfindungsgemäße Verfahren wird insbesondere durch Verwenden bzw. Bereitstellen einer erfindungsgemäßen Vorrichtung ausgeführt.

Die Erfindung wird nachfolgend in nicht einschränkender Weise anhand der Figuren näher erläutert.
- Fig. 1: zeigt eine erfindungsgemäße Vorrichtung zur Stimulation und Erfassung von distorsiv produzierten otoakustischen Emissionen.
- Fig. 2: zeigt ein ideales Spektrum eines mit der Vorrichtung aus Fig. 1 im äußeren Gehörgang aufgenommenen Schalls.
- Fig. 3: zeigt in Anlehnung an Fig. 2 ein Spektrum, das technisch bedingte Verzerrungsanteile enthält.
- Fig. 4: veranschaulicht in einem schematischen Flussdiagramm mehrere Reaktionsmöglichkeiten der erfindungsgemäßen Vorrichtung.

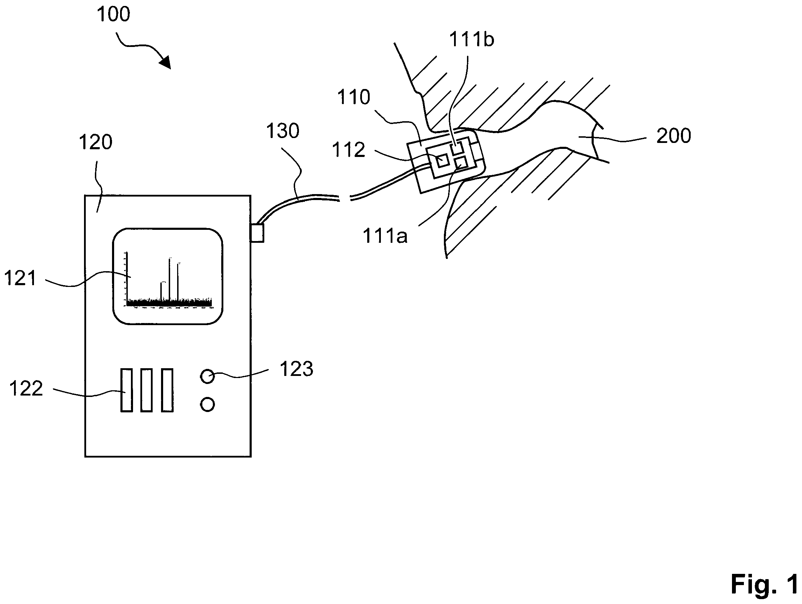

Fig. 1 zeigt eine insgesamt mit 100 bezeichnete Vorrichtung zur Stimulation und Erfassung von distorsiv produzierten otoakustischen Emissionen an einem menschlichen Ohr (oder gegebenenfalls bei entsprechender Ausbildung auch an einem tierischen Ohr). Die Vorrichtung 100 umfasst eine Ohrsonde 110, die gemäß Darstellung dichtend in einem äußeren Gehörgang 200 eingesetzt ist. Die Ohrsonde 110 weist zwei Lautsprecher 111a und 111b zur Erzeugung von Stimulationstönen und ein Mikrofon 112 zur Aufnahme bzw. Registrierung des im äußeren Gehörgang 200 vorhandenen Schalls auf. Die Ohrsonde 110 kann auch nur einen Lautsprecher oder mehr als zwei Lautsprecher aufweisen. Ferner kann die Ohrsonde 110 mehrere Mikrofone aufweisen. Im Übrigen ist denkbar, dass einer oder beide Stimulationstöne nicht in der Ohrsonde 110 sondern außerhalb der Ohrsonde 110 erzeugt wird/werden und durch einen Schlauch (oder dergleichen) oder mechanisch (bspw. als Körper- und insbesondere Knochenschall über den Schädel) in das zu untersuchende Ohr eingeleitet bzw. eingekoppelt wird/werden. D. h. die Ohrsonde 110 käme prinzipiell auch ohne darin eingebaute Lautsprecher 111a und/oder 111b aus, was erfindungsgemäß mit umfasst sein soll. Auch könnten die Lautsprecher und/oder das Mikrofon in verschiedenen Sonden untergebracht werden.

Die Vorrichtung 100 umfasst ferner ein Messgerät 120, das über ein Kabel 130 mit der Ohrsonde 110 verbunden ist und mit dem die Lautsprecher 111a und 111b in der Ohrsonde 110 angesteuert und der vom Mikrofon 112 aufgenommene Schall im Hinblick auf die distorsiv produzierten otoakustischen Emissionen ausgewertet werden kann, wozu das Messgerät 120 eine Auswerteeinrichtung gemäß obenstehenden Erläuterungen umfasst. Das Messgerät 120 weist ein Display 121, mehrere Schalter 122 und mehrere Leuchtanzeigen 123 auf. Die Ohrsonde 110 und das Messgerät 120 können auch als ein Teil ausgebildet sein.

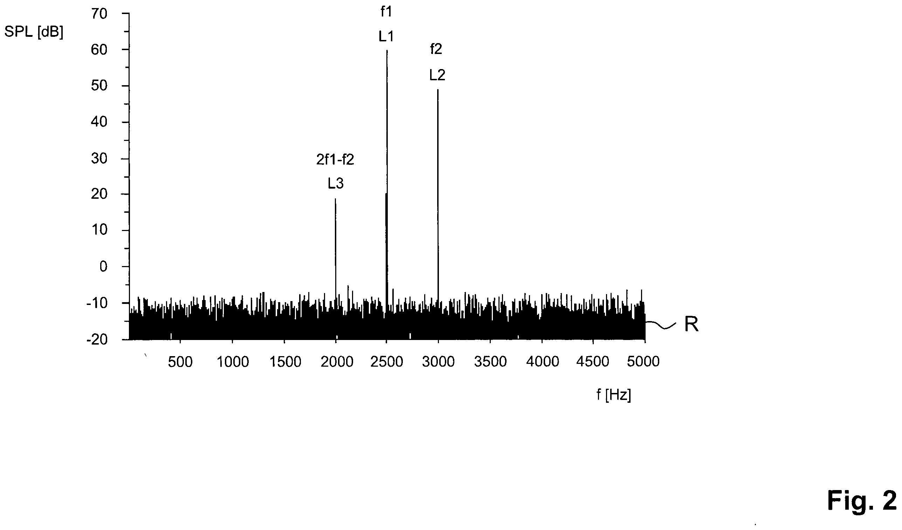

Fig. 2 zeigt beispielhaft ein durch Auswertung im Messgerät 120 erhaltenes Spektrum des im Gehörgang 200 aufgenommenen Schalls. Auf der Abszisse sind die erfassten Frequenzen (in Hz) und auf der Ordinate die zugehörigen Schallpegel (SPL in dB) abgetragen. Dieses Spektrum kann bspw. auf dem Display 121 angezeigt werden.

Bei dem in Fig. 2 dargestellten Spektrum handelt es sich um ein idealisiertes Spektrum, das sich ohne technisch bedingte Verzerrungen ergeben würde (d. h. dieses Spektrum ist frei von technisch bedingten Verzerrungsanteilen). Die mit den Lautsprechern 111a und 111b erzeugten Stimulationstöne bzw. Primärtöne mit den Frequenzen f1 und f2 (wobei gilt: f1 < f2 und insbesondere f2/f1 ≈ 1,2) weisen die Pegel L1 und L2 auf (wobei gilt: L1 ≥ L2; siehe entsprechende Linien im Spektrum). Das Spektrum weist ferner bei der Frequenz (2 . f1) - f2 eine weitere sich über das Rauschen R erhebende Linie auf, die dem maßgeblichen DPOAE-Verzerrungsprodukt (physiologisches Verzerrungsprodukt) bzw. dem Hauptanteil der im Innenohr als Folge der Stimulationstöne erzeugten distorsiv produzierten otoakustischen Emissionen entspricht. Diese typischerweise vorliegende Linie bzw. der entsprechende Pegel L3 kann zur weiteren Auswertung und zur diagnostischen Beurteilung herangezogen werden und ist daher von besonderem Interesse.

Problematisch ist jedoch, dass die technischen Komponenten der Vorrichtung 100, d. h. insbesondere die Lautsprecher 111a und 111b sowie das Mikrofon 112, sowohl beim Erzeugen der Stimulationstöne als auch beim Aufnehmen des Schalls im Gehörgang 200 technisch bedingten Verzerrungen unterliegen können, wobei die hierbei entstehenden Verzerrungsprodukte dann als Verzerrungsanteile auch in das Spektrum eingehen. Derartige Verzerrungen werden im Allgemeinen durch nicht lineare Kennlinieverläufe der technischen Komponenten hervorgerufen, die an irgend einer Stelle nachteilige Auswirkungen haben und zu Verzerrungsprodukten bzw. Verzerrungsanteilen führen. Solche technisch bedingten Verzerrungen bzw. Verzerrungsprodukte sind insbesondere auch in sofern problematisch, als dass der DPOAE-Anteil im aufgenommenen Schall gegenüber den Stimulationstönen oft deutlich schwächer ist. Im ungünstigen Fall kann die Auswertung des aufgenommenen Schalls dann das Vorhandensein bzw. die Anwesenheit von distorsiv produzierten otoakustischen Emissionen ergeben, obwohl tatsächlich keine oder viel schwächere distorsiv produzierten otoakustischen Emissionen vorliegen, oder umgekehrt (d. h. die Auswertung ergibt keine distorsiv produzierten otoakustischen Emissionen obwohl tatsächlich solche vorliegen).

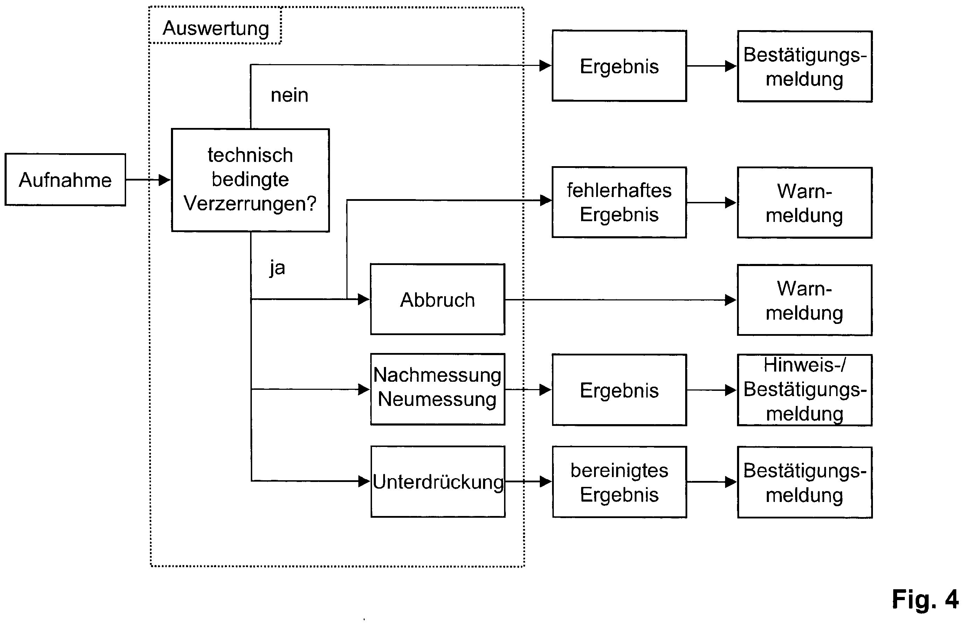

Erfindungsgemäß ist daher vorgesehen, dass das Messgerät 120 der erfindungsgemäßen Vorrichtung 100 dazu ausgebildet ist, das Vorhandensein oder auch Nicht-Vorhandensein von technisch bedingten Verzerrungsprodukten im aufgenommenen Schall automatisch zu erkennen und hierauf zu reagieren. Dieses Erkennen bzw. Nicht-Erkennen von technisch bedingten Verzerrungsprodukten kann insbesondere beim Auswerten des aufgenommenen Schalls erfolgen, wobei diese Auswertung zeitversetzt (insbesondere unter Zwischenspeicherung des aufgenommenen Schalls) oder auch in Echtzeit vorgenommen werden kann. Welche Reaktionen beim Erkennen und/oder Nicht-Erkennen von technisch bedingten Verzerrungsprodukten möglich sind, wird untenstehend anhand der Fig. 4 noch näher erläutert.

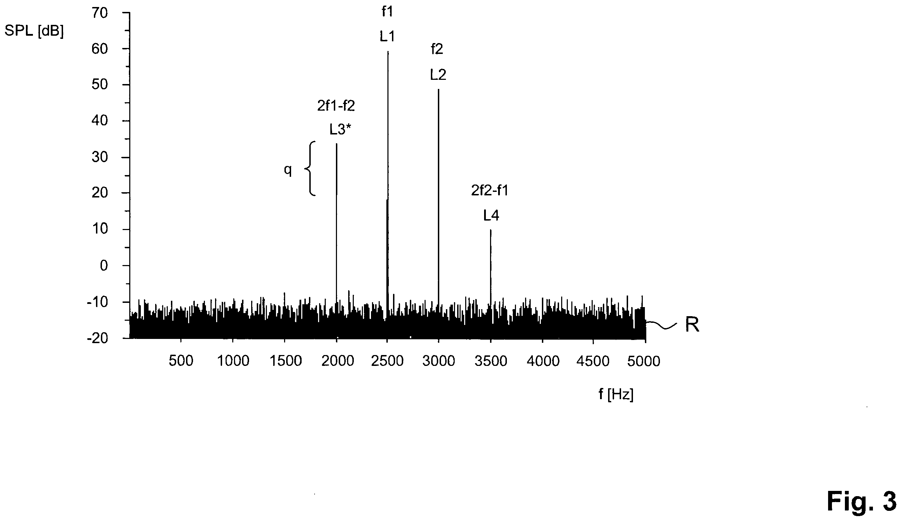

Fig. 3 zeigt analog zur Fig. 2 ein durch Auswertung im Messgerät 120 erhaltenes Spektrum des im Gehörgang 200 aufgenommenen Schalls, wobei der aufgenommene Schall technisch bedingte Verzerrungsprodukte und das daraus abgeleitete Spektrum technisch bedingte Verzerrungsanteile enthält. Die enthaltenen technisch bedingten Verzerrungsanteile führen gegenüber dem in Fig. 2 gezeigten Spektrum zu einem um den Betrag q höheren Pegel L3*. Gegebenenfalls könnte der Pegel L3* aber auch niedriger als der Pegel L3 ausfallen. Beides ist jedoch unerwünscht, da eine zuverlässige diagnostische Beurteilung so nicht möglich ist.

Bei Betrachtung bzw. Analyse des Spektrums bzw. der spektralen Zusammensetzung des aufgenommenen Schalls kann jedoch anhand wenigstens eines insbesondere durch eine Linie repräsentierten zusätzlichen Verzerrungsanteils, bei dem es sich typischerweise nicht um ein physiologisches bzw. biomechanisches Verzerrungsprodukt handelt, auf das Vorhandensein bzw. auf die Anwesenheit von technisch bedingten Verzerrungsprodukten geschlossen werden. D. h. ein solcher "zusätzlicher" Verzerrungsanteil könnte dazu benutzt werden, auf technisch bedingte Verzerrungsprodukte zu schließen, wobei "zusätzlich" bedeutet, dass dieser Verzerrungsanteil in einem idealen Spektrum (wie in Fig. 2 dargestellt) nicht oder mit einem anderen Pegel (Amplitude) und/oder mit einem anderen Pegel relativ zum Hauptanteil der distorsiv produzierten otoakustischen Emissionen vorhanden wäre.

So hat sich z. B. gezeigt (wobei sich selbiges auch durch theoretische Betrachtungen herleiten lässt), dass beim Vorliegen bzw. beim Vorhandensein von technisch bedingten Verzerrungsprodukten eine weitere deutliche Linie im Spektrum auftaucht, nämlich bei der Frequenz (2 . f2) - f1. Diese ausgeprägt in Erscheinung tretende Linie mit dem Pegel L4 kann ein verlässliches Indiz für das Vorhandensein von technisch bedingten Verzerrungsprodukten im aufgenommenen Schall sein.

Zum Hintergrund: Bei der Frequenz (2 · f2) - f1 tritt typischerweise ein Nebenanteil der distorsiv produzierten otoakustischen Emissionen auf, der im Idealfall gegenüber dem Hauptanteil bei (2 · f1) - f2 jedoch deutlich geringer ausfällt. Beim Vorliegen von technisch bedingten Verzerrungsprodukten ist allerdings zu erwarten, dass genau dieser Anteil (bzw. der Pegel L4) deutlich höher (als im Idealfall) ausfällt. Diesen Umstand kann sich die Erfindung zu Nutze machen. In analoger Weise könnten auch die Pegel bei anderen Frequenzen im Spektrum betrachtet werden, bei denen typischerweise Nebenanteile der distorsiv produzierten otoakustischen Emissionen auftreten.

Wird also bei der Auswertung des aufgenommenen Schalls durch Betrachtung bzw. Analyse des Spektrums ein ausgeprägter Pegel L4 bei der Frequenz (2 · f2) - f1 erkannt, so kann hieraus gefolgert werden, dass technisch bedingte Verzerrungsprodukte vorhanden sind. Wird dieser ausgeprägte Pegel nicht erkannt, so kann anderseits hieraus geschlossen werden, dass technisch bedingte Verzerrungsprodukte nicht vorhanden sind. Die Zuordnung kann bspw. durch einen Schwellenwert für den Pegel L4 festgelegt werden. Die Auswertung des aufgenommenen Schalls im Messgerät 120 erfolgt automatisch, wozu dieses mit einem Prozessor bzw. einer prozessorbasierten Recheneinrichtung ausgestattet ist.

Eine weitere Möglichkeit um das Vorliegen bzw. Vorhandensein von technisch bedingten Verzerrungsprodukten zu erkennen und/oder zu verifizieren (gegebenenfalls auch zu falsifizieren) besteht darin, neben der Amplitude der Frequenzlinien im Spektrum auch die Phase der jeweiligen Linie auszuwerten. Diese Phase hängt mit der Laufzeit zusammen, die bei technisch bedingten Verzerrungen anders sein kann als bei distorsiv produzierten otoakustischen Emissionen.

Fig. 4 zeigt in einem schematischen Flussdiagramm mehrere Reaktionsmöglichkeiten, die beim Erkennen von vorhandenen oder auch nicht vorhandenen technisch bedingten Verzerrungsprodukten im aufgenommenen Schall veranlasst bzw. herbeigeführt werden könnten. Diese Reaktionsmöglichkeiten schließen sich nicht grundsätzlich aus, so dass auch mehrere unterschiedliche Reaktionen möglich sind. Bevorzugt ist vorgesehen, dass das Messgerät 120 unterschiedliche Reaktionen herbeiführen kann, die durch den Benutzer voreinstellbar sind, was bspw. anhand der Schalter 122 erfolgen kann.

Werden bei der automatisierten Auswertung des aufgenommenen Schalls keine technisch bedingten Verzerrungsprodukte erkannt bzw. detektiert, so kann die Auswertung fortgesetzt und ein Ergebnis bereitgestellt werden. Das Ergebnisses kann bspw. auf dem Display 121 angezeigt werden. Als Ergebnis der Auswertung kann z. B. das ermittelte Spektrum und/oder der Pegelwert L3 (siehe Fig. 2) angezeigt werden. Die herbeigeführte Reaktion ist als solche für den Benutzer unter Umständen nicht als Reaktion erkennbar. Ergänzend kann daher eine optische und/oder α-kustische Bestätigungsmeldung ausgegeben werden, wozu bspw. auf dem Display 121 eine Bestätigungsmeldung ("Messung O.K.") angezeigt, eine der Leuchtanzeigen 123 (z. B. eine grüne Leuchtdiode) aktiviert und/oder mittels eines im Messgerät 120 verbauten Lautsprechers ein Bestätigungston erzeugt werden kann. Eine Bestätigungsmeldung (oder gegebenenfalls auch Warnmeldung) könnte auch an der Ohrsonde 110 ausgegeben werden, wozu die Ohrsonde 110 mit wenigstens einer Anzeigeeinrichtung wie bspw. einer Leuchtanzeige (z. B. eine Leuchtdiode) und/oder einem zu diesem Zweck vorgesehenen Lautsprecher ausgestattet sein kann. Werden bei der automatisierten Auswertung des aufgenommenen Schalls technisch bedingte Verzerrungsprodukte erkannt, so kann z. B. die Auswertung abgebrochen und eine Warnmeldung kann ausgegeben werden. Bspw. kann auf dem Display 121 eine Warnmeldung ("Messung fehlerhaft") angezeigt, eine der Leuchtanzeigen 123 (z. B. eine rote Leuchtdiode) aktiviert und/oder ein Warnton erzeugt werden. Ebenso ist denkbar, dass trotz erkannter technisch bedingter Verzerrungsprodukte die Auswertung fortgesetzt und ein möglicherweise fehlerbehaftetes Ergebnis bereitgestellt wird, wobei der Benutzer dann durch das Ausgeben einer Warnmeldung bzw. Fehlermeldung entsprechend informiert werden kann.

Ferner besteht die Möglichkeit, dass die Messung bzw. der Messdurchlauf mit geänderten Parametern für die Stimulationstöne (d. h. mit veränderten Frequenzen f1 und/oder f2 und/oder mit veränderten Pegeln L1 und/oder L2) fortgesetzt oder eine neue Messung bzw. ein neuer Messdurchlauf mit geänderten Parametern für die Stimulationstöne gestartet wird, falls technisch bedingte Verzerrungsprodukte erkannt werden. Je nach dem kann die Auswertung unterbrochen oder abgebrochen werden. Das Fortsetzen des Messdurchlaufs ist in Fig. 4 mit "Nachmessung" bezeichnet. Das Ausführen eines neuen Messdurchlaufs ist in Fig. 4 mit "Neumessung" bezeichnet. Ergänzend kann eine Hinweismeldung ausgegeben werden. Bei einem erfolgreichen Abschluss des fortgesetzten bzw. wiederholten Messdurchlaufs, wonach ein zuverlässiges Ergebnis bereitgestellt wird, kann auch eine Bestätigungsmeldung ausgegeben werden.

Eine weitere besonders bevorzugte Reaktion auf das Vorliegen von technisch bedingten Verzerrungsprodukten ist deren Unterdrückung, so dass auf Grundlage des gemessenen Schalls ein bereinigtes bzw. korrigiertes Ergebnis bereitgestellt werden kann. Eine solche Unterdrückung kann bspw. durch eine rechnerische Bereinigung oder dergleichen erfolgen, bei der die technisch bedingten Verzerrungsprodukte quasi herausgerechnet werden, so dass ein bereinigtes bzw. korrigiertes Ergebnis bereitgestellt werden kann. Bezüglich dem in Fig. 3 gezeigten Spektrum könnte dies z. B. bedeuten, dass der Pegel L3* bei der Frequenz (2 · f1) - f2 (bei der an und für sich der maßgebliche DPOAE-Anteil vorliegt) rechnerisch um den zuvor bestimmten Betrag q auf den tatsächlichen Pegel L3 (siehe Fig. 2) verringert wird. D. h. zum tatsächlichen Pegel L3 könnte man durch Subtrahieren des Verzerrungsanteils q vom Pegel L3* gelangen. Auch hierbei kann ergänzend eine Hinweismeldung bzw. Bestätigungsmeldung ausgegeben werden.

Eine solche rechnerische Unterdrückung bzw. Bereinigung kann z. B. anhand wenigstens eines hinterlegten mathematischen Modells erfolgen, welches die Nicht-Linearitäten der zumindest relevanten technischen Komponenten abbildet. Eine solche rechnerische Bereinigung könnte alternativ oder ergänzend aber auch durch das Heranziehen von tabellarisch hinterlegten Vergleichswerten und/oder durch den Vergleich mit simulierten Werten (bspw. mit simulierten Spektren, ähnlich dem in Fig. 2 dargestellten) erfolgen. In diesem Fall kann der Betrag q z. B. dadurch bestimmt werden, indem der simulierte Pegel vom gemessenen Pegel subtrahiert wird (oder umgekehrt).

Bevorzugt ist vorgesehen, dass eine Reaktionen beim Erkennen von vorhandenen technisch bedingten Verzerrungsprodukten erst dann herbeigeführt wird, wenn die technisch bedingten Verzerrungsprodukte eine kritische Schwelle erreichen, überschreiten oder unterschreiten.

Die vorausgehend erläuterten Maßnahmen werden von dem Messgerät 120 automatisiert ausgeführt. Insbesondere ist vorgesehen, dass der gesamte Messdurchlauf (vom Start bis zum Ergebnis) automatisiert ausgeführt wird. Denkbar ist jedoch, dass bei der Ausführung Entscheidungsabfragen erzeugt werden, die vom Benutzer beantwortet werden müssen. Um dies bewerkstelligen zu können weist das Messgerät 120 wenigstens einen Prozessor sowie auch wenigstens einen Datenträger mit einem darauf abgespeicherten ausführbaren Programmcode auf. Ferner kann das Messgerät 120 auch wenigstens eine Speichereinrichtung (bspw. für die Zwischenspeicherung des aufgenommenen Schalls oder zur Speicherung von Zwischen- und/oder Endergebnissen), wenigstens eine Computerschnittstelle und dergleichen mehr aufweisen.

## Patentansprüche

1. Vorrichtung (100) zur Erfassung von distorsiv produzierten otoakustischen Emissionen, insbesondere an einem menschlichen Ohr, umfassend:
- wenigstens eine Ohrsonde (110), die in den äußeren Gehörgang (200) eingesetzt oder an den äußeren Gehörgang (200) angesetzt werden kann und mit der der im äußeren Gehörgang (200) vorhandene Schall aufgenommen werden kann; und
- wenigstens eine Auswerteeinrichtung, mit der der mit der Ohrsonde (110) aufgenommene Schall im Hinblick auf distorsiv produzierte otoakustische Emissionen ausgewertet werden kann,
**dadurch gekennzeichnet, dass**
die Auswerteeinrichtung die Fähigkeit aufweist, im aufgenommenen Schall das Vorhandensein und/oder das Nicht-Vorhandensein technisch bedingter Verzerrungsprodukte, welche fälschlicherweise als das Ergebnis von otoakustischen Emissionen interpretiert werden können, automatisch zu erkennen und hierauf zu reagieren.

2. Vorrichtung (100) nach Anspruch 1,
**dadurch gekennzeichnet, dass**
die Reaktion beim Erkennen von technisch bedingten Verzerrungsprodukten das Ausgeben einer Warnmeldung ist.

3. Vorrichtung (100) nach Anspruch 1,
**dadurch gekennzeichnet, dass**
die Reaktion beim Erkennen von technisch bedingten Verzerrungsprodukten das Fortsetzen des Messdurchlaufs oder Wiederholen des Messdurchlaufs mit geänderten Parametern ist.

4. Vorrichtung (100) nach Anspruch 1,
**dadurch gekennzeichnet, dass**
die Reaktion beim Erkennen von technisch bedingten Verzerrungsprodukten das Unterdrücken dieser technisch bedingten Verzerrungsprodukte ist.

5. Vorrichtung (100) nach Anspruch 4,
**dadurch gekennzeichnet, dass**
in der Auswerteeinrichtung ein mathematisches Modell für technische Komponenten der Vorrichtung (100) hinterlegt ist, welches deren Nicht-Linearitäten abbildet und hiermit die rechnerische Unterdrückung der technisch bedingten Verzerrungsprodukte ermöglicht.

6. Vorrichtung (100) nach einem der vorausgehenden Ansprüche,
**dadurch gekennzeichnet, dass**
die Auswerteeinrichtung die Fähigkeit aufweist, unterschiedliche Reaktionen herbeiführen zu können, falls technisch bedingte Verzerrungsprodukte im aufgenommenen Schall erkannt und/oder nicht erkannt werden, wobei eine herbeizuführende Reaktion durch Auswahl voreinstellbar ist.

7. Vorrichtung (100) nach einem der vorausgehenden Ansprüche,
**dadurch gekennzeichnet, dass**
die Auswerteeinrichtung die Fähigkeit aufweist, das Vorhandensein technisch bedingter Verzerrungsprodukte durch Analyse des Spektrums des aufgenommenen Schalls zu erkennen.

8. Vorrichtung (100) nach einem der vorausgehenden Ansprüche,
**dadurch gekennzeichnet, dass**
die Auswerteeinrichtung in der Ohrsonde (110) integriert ist.

9. Vorrichtung (100) nach einem der vorausgehenden Ansprüche,
**dadurch gekennzeichnet, dass**
diese einen Datenträger mit einem darauf abgespeicherten Programmcode und einen Prozessor aufweist, um wesentliche Funktionen automatisiert ausführen zu können.

10. Verfahren zur Erfassung von distorsiv produzierten otoakustischen Emissionen und insbesondere zur Stimulation und Erfassung von distorsiv produzierten otoakustischen Emissionen, umfassend die folgenden Schritte:
- gegebenenfalls Stimulation von distorsiv produzierten otoakustischen Emissionen;
- Aufnehmen des im äußeren Gehörgang vorhandenen Schalls;
- Auswerten des aufgenommen Schalls im Hinblick auf distorsiv produzierte otoakustische Emissionen; und
- Herbeiführen einer Reaktion, falls beim Auswerten im aufgenommenen Schall das Vorhandensein und/oder das Nicht-Vorhandensein technisch bedingter Verzerrungsprodukte, welche fälschlicherweise als das Ergebnis von otoakustischen Emissionen interpretiert werden können, erkannt wird.

## Claims

1. Device (100) for detecting distortively produced otoacoustic emissions, especially at a human ear, comprising:
- at least one ear canal probe (110), which can be placed in the external ear canal (200) or applied to the external ear canal (200) and with which the sound present in the external ear canal (200) in the ear can be received; and
- at least one evaluation device, by means of which the sound received by the ear canal probe (110) can be evaluated with regard to distortively produced otoacoustic emissions,
**characterized in that**
the evaluation device has the capability of automatically detecting and responding to the presence and/or the absence of technically induced distortion products in the received sound, which can be incorrectly interpreted as the result of otoacoustic emissions.

2. Device (100) according to Claim 1,
**characterized in that**
the response upon detecting technically induced distortion products is the output of a warning message.

3. Device (100) according to Claim 1,
**characterized in that**
the response upon detecting technically induced distortion products is the continuation of the measurement cycle or the repetition of the measurement cycle with changed parameters.

4. Device (100) according to Claim 1,
**characterized in that**
the response upon detecting technically induced distortion products is the suppression of said technically induced distortion products.

5. Device (100) according to Claim 4,
**characterized in that**
a mathematical model for technical components of the device (100) is stored in the evaluation device, which model maps the non-linearities thereof and thus enables the computational suppression of the technically induced distortion products.

6. Device (100) according to one of the preceding claims,
**characterized in that**
the evaluation device has the ability to trigger different responses should technically induced distortion products be detected and/or not be detected in the received sound, wherein a response to be triggered can be present by selection.

7. Device (100) according to one of the preceding claims,
**characterized in that**
the evaluation device has the ability to detect the presence of technically induced distortion products by analyzing the spectrum of the received sound.

8. Device (100) according to one of the preceding claims,
**characterized in that**
the evaluation device is integrated in the ear canal probe (110).

9. Device (100) according to one of the preceding claims,
**characterized in that**
said device includes a data carrier with a program code stored thereon and a processor in order to be able to execute essential functions automatically.

10. Method for detecting distortively produced otoacoustic emissions and in particular for stimulating and detecting distortively produced otoacoustic emissions comprising the following steps:
- optional stimulation of distortively produced otoacoustic emissions;
- receiving the sound present in the external ear canal;
- evaluating the received sound with respect to distortively produced otoacoustic emissions; and
- triggering a response if, during evaluation, the presence and/or absence of technically induced distortion products is detected in the received sound, which distortion products can be incorrectly interpreted as the result of otoacoustic emissions.

## Revendications

1. Dispositif (100) servant à détecter des émissions otoacoustiques produites de manière distorsive, en particulier au niveau d'une oreille humaine, comprenant :
- au moins une sonde d'oreille (110), qui peut être insérée dans le conduit auditif extérieur (200) ou peut être placée au niveau du conduit auditif extérieur (200) et avec laquelle les bruits présents dans le conduit auditif extérieur (200) peuvent être enregistrés ; et
- au moins un système d'évaluation, avec lequel les bruits enregistrés avec la sonde d'oreille (110) peuvent être évalués quant à des émissions otoacoustiques produites de manière distorsive,
**caractérisé en ce que**
le système d'évaluation présente la capacité d'identifier automatiquement dans les bruits enregistrés la présence et/ou l'absence de produits de distorsion d'origine technique, lesquels peuvent être interprétés à tort comme le résultat d'émissions otoacoustiques, et à y répondre.

2. Dispositif (100) selon la revendication 1,
**caractérisé en ce que**
la réponse lors de l'identification de produits de distorsion d'origine technique est l'envoi d'un message d'avertissement.

3. Dispositif (100) selon la revendication 1,
**caractérisé en ce que**
la réponse lors de l'identification de produits de distorsion d'origine technique est la poursuite du cycle de mesure ou la répétition du cycle de mesure avec des paramètres modifiés.

4. Dispositif (100) selon la revendication 1,
**caractérisé en ce que**
la réponse lors de l'identification de produits de distorsion d'origine technique est la suppression desdits produits de distorsion d'origine technique.

5. Dispositif (100) selon la revendication 4,
**caractérisé en ce que**
un modèle mathématique pour des composants techniques du dispositif (100) est enregistré dans le système d'évaluation, lequel reproduit leurs non-linéarités et permet ainsi la suppression arithmétique des produits de distorsion d'origine technique.

6. Dispositif (100) selon l'une des revendications précédentes,
**caractérisé en ce que**
le système d'évaluation présente la capacité de pouvoir entraîner différentes réponses si des produits de distorsion d'origine technique sont identifiés et/ou ne sont pas identifiés dans les bruits enregistrés, dans lequel une réponse à entraîner peut être réglée au préalable par sélection.

7. Dispositif (100) selon l'une des revendications précédentes,
**caractérisé en ce que**
le système d'évaluation présente la capacité d'identifier la présence de produits de distorsion d'origine technique par l'analyse du spectre des bruits enregistrés.

8. Dispositif (100) selon l'une des revendications précédentes,
**caractérisé en ce que**
le système d'évaluation est intégré dans la sonde d'oreille (110).

9. Dispositif (100) selon l'une des revendications précédentes,
**caractérisé en ce que**
celui-ci présente un support de données avec un code de programme sauvegardé sur celui-ci et un processeur pour pouvoir exécuter de manière automatisée des fonctions essentielles.

10. Procédé servant à détecter des émissions otoacoustiques produites de manière distorsive et en particulier servant à stimuler et détecter des émissions otoacoustiques produites de manière distorsive, comprenant les étapes suivantes :
- éventuellement la stimulation d'émissions otoacoustiques produites de manière distorsive ;
- l'enregistrement des bruits présents dans le conduit auditif extérieur ;
- l'évaluation des bruits enregistrés quant à des émissions otoacoustiques produites de manière distorsive ; et
- l'entraînement d'une réponse si, lors de l'évaluation, la présence et/ou l'absence de produits de distorsions d'origine technique, lesquels peuvent être interprétés à tort comme le résultat d'émissions otoacoustiques, sont identifiées dans les bruits enregistrés.
